# EUROPEAN PATENT SPECIFICATION

(11) **EP 2 254 559 B1**
(45) Date of publication and mention of the grant of the patent: **10.10.2018**
(21) Application number: 09708787.8
(22) Date of filing: 05.02.2009
(51) Int. Cl.: A61K 9/50, A61K 47/30, A61K 38/26

(54) **COMPOSITION FOR SUSTAINED RELEASE DELIVERY OF PROTEINS OR PEPTIDES**
ZUSAMMENSETZUNG ZUR VERZÖGERTEN FREISETZUNG VON PROTEINEN ODER PEPTIDEN
COMPOSITION POUR UNE ADMINISTRATION À LIBÉRATION PROLONGÉE DE PROTÉINES OU DE PEPTIDES

(30) Priority: 08.02.2008 US 65179
(43) Date of publication of application: 01.12.2010
(73) Proprietor: Foresee Pharmaceuticals Co., Ltd., Taipei City 115 (TW)
(72) Inventor: LI, Yuhua, Newark DE 19713 (US); GUARINO, Andrew, Newark DE 19702 (US); CHIEN, Benjamin, Woodside, CA 94062 (US)
(74) Representative: Gulde & Partner
(86) International application number: PCT/US2009/033217
(87) International publication number: WO 2009/100216

(56) References cited:
- US-A- 5 747 058
- US-A- 6 110 503
- US-A1- 2006 025 328
- US-A1- 2006 210 599
- US-A1- 2008 020 016
- US-A1- 2008 020 016
- US-B1- 6 239 107
- US-B2- 7 244 709

## Description

### 1. Field of the Invention

This invention relates to the field of sustained release delivery of proteins or peptides and to compositions and methods useful for sustained release delivery of proteins or peptides that are covalently modified and formulated with a hydrophobic, non-polymeric carrier material.

### BACKGROUND OF THE INVENTION

Hydrophobic, non-polymeric materials, particularly, highly viscous, non-polymeric liquid materials have been described as biodegradable systems for controlled release delivery of bioactive compounds (Smith and Tipton, Pharmaceutical Research, 13(9), S300, 1996). The hydrophobic non-polymeric material is generally substantially insoluble in water. The hydrophobic non-polymeric material can be a highly viscous liquid that has a viscosity of at least 5,000 cP at 37° C and does not crystallize neat under ambient or physiological conditions. When such material is mixed with a small amount of plasticizing solvent, the mixture has a much lower viscosity than that of the non-polymeric liquid material alone. This low viscosity solution can be easily formulated with a bioactive compound and the resulting low viscosity liquid formulation can be readily administered to the body of a subject to form a highly viscous depot *in-situ.*

Representative examples of such *in-situ* forming depot systems containing the hydrophobic, non-polymeric liquid carrier materials are disclosed in the U.S. Pat. Nos. 5,747,058; 5,968,542; 6,051,558; and 6,992,065 as well as US application US 2008/0020016 A1. The compositions described in these patents comprise a hydrophobic, highly viscous, non-polymeric liquid material such as sucrose acetate isobutyrate (SAIB), a water soluble or miscible organic solvent, and a bioactive substance. Such a composition can be easily prepared and administered to the body of a subject in the form of a low viscosity solution. Once in the body, the solvent dissipates or diffuses into the surrounding tissues, which leads to the precipitation or coagulation of the non-polymeric materials to form a highly viscous gel, semi-solid, or solid depot that encapsulates the bioactive substances. Then the bioactive substance is released via dissolution, diffusion, and/or degradation of the depot.

However, due to the hydrophobic nature of the non-polymeric carrier material, many bioactive agents, especially hydrophilic peptides and proteins with their charged and polar characteristics, may not be compatible with the non-polymeric carrier material, resulting in an unstable liquid formulation. It has been found that the composition comprising a hydrophobic non-polymeric liquid material, a peptide, and an organic solvent undergoes phase separation when it is allowed to stand for a short period of time at ambient condition. In addition, the proteins or peptides, due to their neucleophilic nature, may interact or react with the non-polymeric carrier materials in solution/suspension of an organic solvent. The interactions/reactions will result in the expedited degradation of the non-polymeric carrier material and can also alter the proteins or peptides chemically. The instability of the carrier material and the protein or peptide in the formulation prohibits the preparation of a suitable composition with a reasonable period of time for storage and the use of the formulation to form a consistent depot upon administration to achieve desired release characteristics. Furthermore, a burst release is the typical characteristic of this type of liquid formulation. The uncontrollable initial burst may not be desirable, especially for the proteins or peptides having a narrow therapeutic index. Although some polymeric additives were disclosed to modulate the release rate in such formulations, the results obtained were not entirely satisfactory.

Therefore, there is a need for a composition that solves these problems and that is useful in the sustained release delivery of a therapeutically effective amount of proteins or peptides over a long period of time.

### SUMMARY OF THE INVENTION

The present invention provides a novel liquid composition suitable for *in-situ* formation of a depot system to deliver a protein or peptide in a controlled, sustained manner. The composition of the present invention is defined in the claims and comprises: (a) a hydrophobic non-polymeric carrier material as defined in the claims; (b) a water miscible biocompatible organic solvent having a miscibility as defined in the claims that dissolves the hydrophobic non-polymeric material and lowers the viscosity of the composition significantly to facilitate the ease of preparation and administration; and (c) a protein or peptide covalently conjugated with amphiphilic molecule(s) as defined in the claims. Wherein the non-polymeric material is substantially insoluble in water and may be a highly viscous liquid that has a viscosity of at least 5,000cP at 37°C and does not crystallize neat under ambient or physiological conditions. The composition of the present invention further comprises optionally an additive to achieve desired release characteristics. The present invention also provides a method of manufacturing and use of the composition thereof.

Accordingly, the protein or peptide is preferably covalently conjugated with a formulation performance-enhancing compound that stabilizes the protein or peptide, increases the compatibility with non-polymeric carrier material, and improves the release profile of the protein or peptide. A suitable formulation performance-enhancing compound is either hydrophilic, lipophilic, or amphiphilic. The compound may be a small molecule or a polymer. The suitable formulation performance-enhancing compound is conjugated to a protein or peptide through a degradable or non-degradable bond. The resulting conjugate preferably retains some or all of the native biological activities of the unmodified protein or peptide.

Then the conjugated protein or peptide is mixed with or dispersed in the hydrophobic, non-polymeric carrier materials. Alternatively, the hydrophobic, non-polymeric carrier material is preferably mixed with a water soluble or miscible solvent such as N-methyl-2-pyrrolidone (NMP) or ethanol to form a low viscosity solution. Then this low viscosity solution is used to dissolve or suspend the conjugated protein or peptide to form a homogeneous solution or uniform suspension. Typically, such formulation containing an unconjugated protein or peptide or its simple salt, such as acetate salt, undergoes rapid phase separation. However, it has been surprisingly discovered that the conjugation of proteins or peptides with formulation performance-enhancing compound of the present invention through covalent bonding can prevent the phase separation to maintain the physical stability of the formulation. In addition, typically the uncomplexed protein or peptide or its simple salt such as acetate salt is susceptible to chemical degradation during formulation process and subsequent storage. It has also been found that such chemical degradation can be prevented or minimized by covalent conjugation of the protein or peptide with the formulation performance-enhancing compound of the present invention. The enhanced chemical and physical stabilities of the composition of the present invention will allow one to develop a stable product with a desired characteristics and a reasonable storage shelf life.

When the liquid composition of the present invention is brought in contact with an aqueous environment, such as biological fluids in the body of a subject, the water soluble or miscible solvent dissipates or diffuses into the surrounding aqueous or biological fluids. Simultaneously, the water insoluble, non-polymeric carrier material precipitates or coagulates to form a highly viscous gel, semi-solid, or solid depot that traps or encapsulates the protein or peptide. Due to the rapid diffusion of the solvent, typically a high initial burst release of the protein or peptide is observed during the depot formation process. However, it has been unexpectedly found that the conjugation of proteins or peptides with formulation performance-enhancing compound through covalent bonding, dramatically reduces the burst effect and improves the overall release profile of the protein or peptide relative to the formulation containing unconjugated protein or peptide. Once the depot is formed, the protein or peptide is released from the non-polymeric matrix by dissolution, diffusion and/or degradation of the non-polymeric carrier material.

According to the present invention, the composition optionally includes additives that modify the composition to achieve a desired release profile for the protein or peptide. The additives include, but are not limited to, burst effect reducing materials, release rate modulating agents, pH modifiers, antioxidants, solubilization agents and the like. The additives can be polymeric or non-polymeric materials including biodegradable or non-biodegradable polymers, carbohydrates or carbohydrate derivatives, organic or inorganic compounds.

The composition of the present invention may be a viscous or non-viscous liquid, or gel that may be administered using a syringe or similar devices. The composition can be administered by injection subcutaneously, intramuscularly, intraperitoneally, or intradermally to form a depot *in-situ.* The compositions can also be administered orally or topically. When administered to the body of a subject, the controlled release of the protein or peptide can be sustained for a desired period of time depending upon the make up of the composition. With the proper selections of the non-polymeric carrier material and other excipients, the duration of the sustained release of the protein or peptide can be controlled over a period of time from several days, to weeks, to one year.

Other objects and features of the present invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not necessarily drawn to scale and that, unless otherwise indicated, they are merely intended to conceptually illustrate the structures and procedures described herein.

### BRIEF DESCRIPTION OF THE DRAWINGS

**In the drawings:**
Figure 1 shows the pictures taken after the formulations stood at room temperature for 24 h.
Figure 2. shows the *in vitro* release of octreotide from SAIB/NMP formulations containing (a) OCT-Ac; (b) PAL-PEG-BA-OCT

### DETAILED DESCRIPTION OF THE PRESENTLY PREFERRED EMBODIMENTS

The present invention is related to the surprising discovery that the covalent modification of a protein or peptide with a formulation performance-enhancing compound enables the preparation of a stable formulation of the protein or peptide in hydrophobic, non-polymeric materials, prevents or minimizes the degradation/reaction of the protein or peptide during formulation process and subsequently storage, and improves the sustained release profile of the protein or peptide. The surprising discovery leads to the development of desired compositions suitable for *in-situ* formation of a depot system to deliver proteins or peptides in a sustained and controlled manner.

The present invention provides a composition as defined in the claims, comprising: (a) a hydrophobic, non-polymeric carrier material as defined in the claims; (b) a water miscible pharmaceutically acceptable solvent as defined in the claims; and (c) a protein or peptide covalently conjugated with amphiphilic molecule(s) as defined in the claims. The composition of the present invention optionally further comprises an additive to achieve desired release characteristics. The present invention also provides a method of manufacturing and use of the composition thereof.

The hydrophobic, non-polymeric material is used as a carrier to control the sustained release of the proteins or peptides. Numerous pharmaceutically acceptable non-polymeric materials are available to a person in the art to produce suitable pharmaceutical compositions that can be used for sustained release delivery of various proteins or peptides. Representative examples of patents relating to hydrophobic, non-polymeric delivery systems and the preparation include U.S. Pat. Nos. 5,736,152; 5,888,533; 6,120,789; 5,968,542; and 5,747,058.

Suitable non-polymeric carrier materials include, but are not limited to, cholesteryl esters such as cholesteryl stearate; C16-C32 mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didocosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan monopalmitate and sorbitan tristearate; esters of fatty alcohols and fatty acids such as cetyl palmitate and cetearyl palmitate; phospholipids including phosphatidylcholine (lecithin), phosphatidylserine, phosphatidylethanolamine, phosphatidylinositol, and lysoderivatives thereof; sphingosine and derivatives thereof; spingomyelins such as stearyl, palmitoyl, and tricosanyl spingomyelins; ceramides such as stearyl and palmitoyl ceramides; glycosphingolipids; and combinations and mixtures thereof.

Preferred non-polymeric carrier materials are those that have low crystallinity, non-polar characteristics, and are hydrophobic. More preferably, the non-polymeric carrier material is a viscous liquid. The non-polymeric liquid material is preferably hydrophobic, substantially water insoluble and has a viscosity of at least 5,000 cP at 37°C that does not crystallize neat under ambient or physiological conditions. As used herein, the term "hydrophobic or water-insoluble" refers to the solubility of a material in water at 25°C is less than one percent by weight. The term "non-polymeric" refers to esters or mixed esters having essentially no repeating units in the acid moiety used to form the esters. The acid moiety used to form the ester or mixed esters may include a small number of repeating units (*i.e.,* oligomers) such as dimers, trimers, tetramers, and pentamers. Generally, the repeating units in acid moiety should be less than five.

Particularly, the hydrophobic, non-polymeric carrier materials can be one or more of non-polymeric esters or mixed esters. The esters are typically formed from a polyol having less than 20 hydroxyl groups that are esterified with carboxylic acids. Suitable polyols include monofunctional and multifunctional alcohols having from 2 to 24 carbons, sugar alcohols, monosasaccharides, disacchrides, oligosacchrides, and polyether alcohols. More specifically, the polyols may be dodecanol, hexanediol, glycerol, mannitol, sorbitol, glucose, fructose, sucrose, inositol, polyglycerol, polyethylene glycol, polypropylene glycol, poly(ethylene-co-propylene) glycol, polyvinyl alcohol, and the like.

The carboxylic acids used to form the hydrophobic non-polymeric carrier materials include organic acids having more than two carbons, such as fatty acids. These carboxylic acids may be saturated, unsaturated, aromatic (aryl or arylalkyl) and linear or branched in structure. These carboxylic acids may also have one or more hydroxyl groups or other groups such as halo, nitro and the like. More specifically, these carboxylic acids include acetic acid, propionic acid, butyric acid, isobutyric acid, valeric acid, lipoic acid, hexanoic acid, heptanoic acid, oleic acid, palmitic acid, stearic acid, myristic acid, benzoic acid, glycolic acid, lactic acid, □-hydroxycaproic acid, octanoic acid, decanoic acid, dodecanoic acid, tetradecanoic acid, hexadecanoic acid, octadecanoic acid, eicosanoic acid, docosanoic acid, and other fatty acids.

The hydrophobic, non-polymeric carrier material is preferably biodegradable without the generation of any non-biocompatible or toxic metabolites. When the hydrophobic, non-polymeric carrier material is mixed with a water miscible solvent, a solution with significant lower viscosity can be obtained. The low viscosity of the solution can be readily combined with a protein or peptide to prepare the composition of the present invention. The low viscosity also allows the composition to be easily administered to the body of a subject. The ratio of the hydrophobic, non-polymeric carrier material to solvent can be easily adjusted to obtain a desired viscosity.

In a preferred embodiment, sucrose acetate isobutyrate (SAIB) is used as a hydrophobic, non-polymeric carrier material. The SAIB is a mixed ester of sucrose esterified with two acetate and six isobutyrate groups. The ester is completely non-crystalline and has a viscosity of over 100,000 cP at 30°C. The viscosity of the ester can be dramatically decreased by slightly increasing the temperature or adding solvents. The SAIB is commercially available from Eastman Chemical Company, USA and may be synthesized following the procedures described in U.S. Pat. No 2,931,802. In one embodiment, the SAIB can be heated and mixed with a protein or peptide covalently conjugated with at least one formulation performance-enhancing compound to prepare a suspension.
Alternatively, the SAIB can be mixed with a large number of biocompatible solvents to result in a low viscosity solution that can be easily formulated with a protein or peptide to obtain an injectable solution or suspension.

Suitable solvents for optional use in the composition of the present invention are biocompatible and water soluble or miscible to dispersible. The solvents have a solubility of at least 1%, preferably have a solubility of at least 3%, more preferably have a solubility of at least 7% by weight in water at 25°C. When combined with the hydrophobic, non-polymeric carrier material, the solvents can dramatically reduce the viscosity of the mixture relative to the non-polymeric carrier material alone. Such lower viscosity liquid composition can be further formulated with a protein or peptide for sustained release delivery. Examples of the suitable solvents, without limitation, include acetone, benzyl alcohol, butylene glycol, caprolactam, caprolactone, dimethylsulfoxide (DMSO), ethanol, ethyl acetate, ethyl lactate, glycerol, glycerol formal, glycofurol (tetraglycol), N-methyl-2-pyrrolidone (NMP), polyethylene glycol, PEG-300, PEG-400, methoxy polyethylene glycol, mPEG-350, alkoxy polyethylene glycol, propylene carbonate, 2-pyrrolidone, triacetin, triethyl citrate, and combinations thereof.

As used herein, peptides are short polymers formed from the linking, in a defined order, of α-amino acids. The link between one amino acid residue and the next is known as an amide bond or a peptide bond. Proteins are polypeptide molecules (or consist of multiple polypeptide subunits). The distinction is that peptides are short and polypeptides/proteins are long. As used herein, peptides, polypeptides, and proteins are used interchangeably and represent the same type of molecules.

The suitable bioactive proteins or peptides of the present invention include any proteins or peptides that have at least one functional group that can be covalently modified and retain some or all of their biological activities. The proteins or peptides of the present invention include, but are not limited to, oxytocin, vasopressin, adrenocorticotropic hormone (ACTH), epidermal growth factor (EGF), platelet-derived growth factor (PDGF), prolactin, luteinising hormone, luteinizing hormone releasing hormone (LHRH), LHRH agonists, LHRH antagonists, growth hormones (including human, porcine, and bovine), growth hormone releasing factor, insulin, insulin-like growth factors (IGF-I, IGF-II), erythropoietin (including all proteins with erythropoietic activity), somatostatins, glucagon, interleukin, interferon-α, interferon-β, interferon-γ, gastrin, tetragastrin, pentagastrin, urogastrone, secretin, calcitonin, enkephalins, endorphins, angiotensins, thyrotropin releasing hormone (TRH), tumor necrosis factor (TNF), parathyroid hormone (PTH), nerve growth factor (NGF), granulocyte-colony stimulating factor (G-CSF), granulocyte macrophage-colony stimulating factor (GM-CSF), macrophage-colony stimulating factor (M-CSF), heparinase, vascular endothelial growth factor (VEG-F), bone morphogenic protein (BMP), hANP, glucagon-like peptide (GLP-1, GLP-2), exenatide (exendin-3, exendin-4, etc.), peptide YY (PYY), renin, bradykinin, bacitracins, polymyxins, colistins, tyrocidine, gramicidins, cyclosporins (which includes synthetic analogues and pharmacologically active fragments thereof), enzymes, cytokines, antibodies, vaccines, antibiotics, glycoproteins, follicle stimulating hormone, kyotorphin, taftsin, thymopoietin, thymosin, thymostimulin, thymic humoral factor, serum thymic factor, colony stimulating factors, motilin, bombesin, dinorphin, neurotensin, cerulein, urokinase, kallikrein, substance P analogues and antagonists, angiotensin II, blood coagulation factor VII and IX, lysozyme, gramicidines, melanocyte stimulating hormone, thyroid hormone releasing hormone, thyroid stimulating hormone, pancreozymin, cholecystokinin, human placental lactogen, thrombopoietin (TPO), human chorionic gonadotrophin, protein synthesis stimulating peptide, gastric inhibitory peptide, vasoactive intestinal peptide, platelet derived growth factor, and synthetic analogues and modifications and pharmacologically-active fragments thereof.

In a preferred aspect, the protein or peptide is covalently conjugated with a formulation performance-enhancing compound. The covalently conjugated protein or peptide retains some or all the biological activities and enhances the formulation performance of the parent agent. The "formulation performance-enhancing compound" as used herein is one that can be covalently conjugated to the protein or peptide and the resulting conjugate substantially retains at least some of the biological activities of the protein or peptide. The conjugated protein or peptide is easier to formulate with the non-polymeric carrier materials and the resulting formulation is more uniform and stable relative to that obtained using non-conjugated protein or peptide. The conjugation enhances the physico-chemical stability and improves the release profile of the protein or peptide from the delivery systems comprising the hydrophobic, non-polymeric carrier materials. The covalent modification of the protein or peptide may also lead to extended *in vivo* disposition half-life, reduced antigenicity and immunogenicity, resistance to proteolysis, improved bioavailability, as well as reduced toxicity.

As used herein, the term "formulation performance-enhancing compound" refers to any molecules after covalent conjugations that improve the formulation performance of the protein or peptide in the non-polymeric carrier material of the present invention. The compound may be hydrophilic, lipophilic, or amphiphilic. The compound may be a small molecule or a polymer. The criterion for the formulation performance-enhancing compound is to retain some or all the biological activities of the protein or peptide after covalent modification and to improve the performance characteristics when formulated with the non-polymeric carrier material of the present invention. The conjugation of the formulation performance-enhancing compound of the present invention with protein or peptide retains at least 10% of the original biological activity of the protein or peptide, preferably at least 25% of the original biological activity of the protein or peptide, more preferably at least 50% of the original biological activity of the protein or peptide. The conjugation of the formulation performance-enhancing compound of the present invention with protein or peptide improves the physical and chemical stabilities of the formulation, while reducing the initial burst release.

In a preferred embodiment, the formulation performance-enhancing compound refers to any hydrophilic polymers. The hydrophilic polymers are water-soluble and can be a linear or branched polymer. Representative examples, but are not limited to, include polyethylene glycol, polypropylene glycol, polyvinylpyrrolidone, polysaccharides, sugar, and the like. Preferably, the molecular weight of the polymer ranges from about 200 daltons to about 50,000 daltons. Hydrophilic polymers for use in the present invention typically have at least one reactive group that can be used to covalently conjugate to the protein or peptide of interest through amino, carboxyl, sulfhydryl, phosphate or hydroxyl functional groups. Various methods of conjugations and pegylations are disclosed in the prior art (U.S. Pat. Nos. 4,179,337; 5,446,090; 5,880,255; and 6,113,906; M.J. Roberts, M.D. Bentley and J.M. Harris, Chemistry for peptide and protein PEGylation, Advanced Drug Delivery Reviews, 2002, 54 (4), 459-476. F.M. Veronese, Peptide and protein PEGylation: a review of problems and solutions, Biomaterials, 2001, 22, 405-417).

In another preferred embodiment, the formulation performance-enhancing compound is a hydrophobic or lipophilic molecule. Typically, the lipophilic molecules have a solubility in water at 20°C less than 1% by weight. The suitable lipophilic formulation performance-enhancing compound is preferably selected from C₄₋₃₆-alkyl, C₄₋₃₆-alkenyl, C₄₋₃₆-alkadienyl, tocopherol and steroidal residues. The terms "C₄₋₃₆-alkyl", "C₄₋₃₆-alkenyl" and "C₄₋₃₆-alkadienyl" are intended to cover straight chain and branched, preferably straight chain, saturated, monounsaturated and di-unsaturated hydrocarbon of 4-36 carbon atoms. Preferably, the lipophilic molecules have high affinity to the cell membrane and can interact with plasma proteins such as albumin to extend the *in vivo* half-life of the modified protein or peptide relative to the unmodified protein or peptide. More specifically, the lipophilic formulation performance-enhancing compound is a saturated or unsaturated hydrocarbyl or carboxylic acyl radical having at least 4 carbon atoms. The carboxylic acyl radical may be caproyl, laurly, palmitoyl, stearoyl, oleyl, eicosanoyl, and docsanoyl. The hydrocarbyl may be hexyl, dodecyl, and octadecyl.

In yet another preferred embodiment, the formulation performance-enhancing compound includes any amphiphilic molecules. The term "amphiphilic" refers to any molecules having both lipophilic and hydrophilic characteristics and soluble in both water and lipophilic solvents. The amphiphilic molecules used in the present invention are composed of lipophilic and hydrophilic moieties. The lipophilic moieties are preferably natural fatty acids or alkyl chains as those described above. The hydrophilic moieties are selected from polyethylene glycol, polypropylene glycol, copolymer of ethyleneglycol and propyleneglycol, polyvinylpyrrolidone, polysaccharides, sugar, and the like. The hydrophilic moieties are preferably polyethylene glycol (PEG) having less than 1000 ethylene glycol units. The size and composition of the lipophilic moieties and the hydrophilic moieties may be adjusted to obtain desired amphiphilicity.

The covalent conjugation of a formulation performance-enhancing compound to a protein or peptide may lead to an improved therapeutic effect compared with the native protein or peptide. The conjugation can typically be done by reaction of a functional group such as an amine group in a bioactive molecule with an acid or other reactive groups in a formulation performance-enhancing compound. Alternatively, the conjugation between a protein or peptide and a formulation performance-enhancing compound is accomplished through an additional moiety such as a bridge, spacer, or linkage moiety, which can be degradable or non-degradable. Representative examples are disclosed in the prior art [*e.g.,* Japanese patent application 1,254,699; U.S. Pat. No. 5,693,609, WO95/07931, U.S. Pat. No. 5,750,497, and WO96/29342. See also, Hashimoto, M., et al., Pharmaceutical Research, 6:171-176 (1989), and Lindsay, D. G., et al., Biochemical J., 121:737-745 (1971)]. Further examples of acylated peptides are found in WO98/08871, WO98/08872, and WO99/43708.

In one embodiment of the present invention, palmitic acid was activated with N-hydroxysuccinimide and then reacted with amine groups on octreotide, an octapeptide, to form a conjugate through an amide linker between the palmityl lipophilic moiety and the peptide. There are two primary amine groups on octreotide. Both amine groups could be conjugated simultaneously or only one amine group could be selectively conjugated by adjusting the reaction conditions followed by separation.

In another embodiment, decanal, a lipophilic compound with an aldehyde end group, was reacted with the amine groups on octreotide to form a conjugate through a secondary amine linkage. Both amine groups could be conjugated simultaneously or only one amine group could be conjugated by adjusting the reaction conditions followed by separation.

In a further embodiment, palmitic acid was conjugated to lysozyme through its six amine groups at several ratios. When the ratio of palmitic acid to lysozyme is smaller than 6, the conjugation sites on lysozyme may be random depending upon the reactivity of each amine group.

According to the present invention, a lipophilic moiety may be first covalently coupled to a hydrophilic moiety to form an amphiphilic molecule. The amphiphilic molecules of the present invention may have one or more suitable functional groups, or may be modified to have one or more suitable functional groups for covalent coupling to a peptide or protein. Suitable functional groups are selected from hydroxyl group, amino group (primary amino group or secondary amino group), thiol group, carboxyl group, aldehyde group, isocynato group, sulfonic acid group, sulfuric acid group, phosphoric acid group, phosphonic acid group, allylic halide group, benzylic halide group, substituted benzylic halide group, and oxiranyl group.

A protein or peptide may be directly or indirectly coupled with one or more amphiphilic moieties through an ester group, amide group, secondary or tertiary amine group, carbamate group, sulfonate group, sulfate group, phosphate group, phosphonate group, or ether group.

Preferably, a protein or peptide is covalently conjugated to one or more amphiphilic molecules comprising (a) a hydrophilic moiety and (b) a lipophilic moiety, wherein the balanced hydrophilic and lipophilic characteristics of the amphiphilic molecule impart the conjugate with suitable solubility in biological fluid or aqueous solution.

More preferably, a protein or peptide is covalently conjugated to one or more amphiphilic molecules comprising (a) a linear polyethylene glycol moiety and (b) a lipophilic moiety, wherein the protein or peptide, the polyethylene glycol and the lipophilic moiety are conformationally arranged to have the lipophilic moiety exteriorly available for interaction with lipophilic environment or cell membranes. Such amphiphilically modified protein or peptide has an enhanced chemical resistance to the reaction with the non-polymeric carrier material both *in vitro* and *in vivo,* relative to the unconjugated protein or peptide.

Preferably, the amphiphilic molecule has the following general structure:

L-S-(OC₂H₄)ₘOH (Formula 1)

wherein L is the lipophilic moiety preferably selected from C₄₋₃₆-alkyl, C₄₋₃₆-alkenyl, C₄₋₃₆-alkadienyl, tocopherol and steroidal residues, and wherein S is a linker selected from a group of an ester group, amide group, secondary or tertiary amine group, carbamate group, sulfonate group, sulfate group, phosphate group, phosphonate group, or ether group, and wherein m ranges from 1 to 1000. Polyethylene glycol (PEG)-lipid conjugates, such as 1,2-Distearoyl-*sn*-Glycero-3-Phosphoethanolamine-N-[Carboxy(Polyethylene Glycol), 1,2-Distearoyl-*sn*-Glycero-3-Phosphoethanolamine-N-[Maleimide(Polyethylene Glycol), 1,2-Distearoyl-*sn-*Glycero-3-Phosphoethanolamine-N-[PDP(Polyethylene Glycol), 1,2-Distearoyl-*sn*-Glycero-3-Phosphoethanolamine-N-[Amino(Polyethylene Glycol), and the like may also be conjugated with proteins or peptides.

In one embodiment, an alkyl group of 16 carbons was covalently coupled to a polyethylene glycol molecule through an ether linkage. The resulting amphiphilic molecule has one hydroxyl group which can be activated or derivatized to react with suitable functional groups on proteins or peptides. In one embodiment of the present invention, the amphiphilic molecule was derivatized to have an aldehyde end group. Then the amphiphilic molecule was covalently conjugated to octreotide through the reaction with amine groups on octreotide followed by reduction reaction with NaCNBH₃. Both amine groups on octreotide could be conjugated simultaneously or only one amine group could be selectively conjugated by adjusting the reaction conditions followed by separation. The conjugate was formed through a secondary amine that does not change the charge characteristics of the unconjugated octreotide. This property may be useful to retain the activity of the protein or peptide.

In another embodiment, the amphiphilic molecule monopalmityl poly(ethylene glycol) (Mn ∼1124) was activated with 4-nitrophenyl chloroformate. Then the amphiphilic molecule was covalently conjugated to octreotide through the reaction with amine groups on octreotide. Both amine groups on octreotide could be conjugated simultaneously or only one amine group could be selectively conjugated by adjusting the reaction conditions followed by separation.

According to the present invention, proteins or peptides covalently modified with one or more formulation performance-enhancing compounds include, for example, pharmaceutically acceptable salts and complexes of the modified protein or peptide. The modification can be at one or more sites on the protein or peptide. Such proteins or peptides also include, for example, site-specifically modified proteins or peptides and mixtures of mono-site and multiple-site modified proteins or peptides.

A "pharmaceutically acceptable salt" means a salt formed between any one or more of the charged groups in a protein or peptide and any one or more pharmaceutically acceptable, non-toxic cations or anions. Organic and inorganic salts include, for example, those prepared from acids such as hydrochloric, sulfuric, sulfonic, tartaric, fumaric, hydrobromic, glycolic, citric, maleic, phosphoric, succinic, acetic, nitric, benzoic, ascorbic, *p*-toluenesulfonic, benzenesulfonic, naphthalenesulfonic, propionic, carbonic, and the like, or for example, ammonium, sodium, potassium, calcium, or magnesium.

According to the present invention, the composition optionally includes additives that modify the composition to achieve desired release profile for the protein or peptide. The additives may be included to modulate release rate and stabilize the protein or peptide. Suitable additives can be any polymeric or non-polymeric materials including biodegradable or non-biodegradable polymers, carbohydrates or carbohydrate derivatives, organic or inorganic compounds. The additives can act as, for example, antioxidants, pH stabilizers, anti-irritants, dispersing agents, bulking agents, binders, and the like.

Some suitable additives are described in U.S. Pat. No. 5,747,058. Preferably, the suitable additives are biocompatible and/or biodegradable polymers. Such polymers include, but are not limited to, polylactides, polyglycolides, polycaprolactones, polyanhydrides, polyamines, polyurethanes, polyesteramides, polyorthoesters, polydioxanones, polyacetals, polyketals, polycarbonates, polyphosphoesters, polyoxaesters, polyorthocarbonates, polyphosphazenes, succinates, poly(malic acid), poly(amino acids), polyvinylpyrrolidone, polyethylene glycol, polyhydroxycellulose, chitin, chitosan, hyaluronic acid and copolymers, terpolymers and mixtures thereof.

According to the present invention, the composition optionally includes reducing agents, antioxidants, and free radical scavengers to stabilize the composition. Examples are, but are not limited to, cysteine or methionine, *d*-alpha tocopherol acetate, *d1*-alpha tocopherol, ascorbyl palmitate, butylated hydroxyanidole, butylated hydroxyanisole, butylatedhydroxyquinone, butylhydroxyanisol, hydroxycomarin, butylated hydroxytoluene, cephalm, ethyl gallate, propyl gallate, octyl gallate, lauryl gallate, propylhydroxybenzoate, trihydroxybutyrophenone, dimethylphenol, ditertbutylphenol, vitamin E, and lecithin.

Accordingly, the composition of the present invention comprises a hydrophobic, non-polymeric carrier material, an optional pharmaceutically acceptable organic solvent, a protein or peptide covalently conjugated with a formulation performance-enhancing compound, and optionally an additive.

The composition of the present invention provides a viscous gel or solid depot for the controlled and sustained release of the protein or peptide when implanted. The controlled release can be sustained for a desired period of time dependent upon the make-up of the composition. With the proper selection of the non-polymeric carrier material and other components, the period of sustained release can be controlled to a desired period of time from a few days to several months.

In a preferred method of preparing and administering the composition of the present invention, the protein or peptide is covalently modified with a formulation performance-enhancing compound. The molar ratio of the protein or peptide to formulation performance-enhancing compound in the conjugate will vary, for example, from 1:1 to 1:10 according to the nature of the protein or peptide. The covalently conjugated protein or peptide may be combined with the hydrophobic, non-polymeric carrier material and optional pharmaceutically acceptable solvent and other optional additives as a single-phase formulation suitable for shelf storage for a reasonable period of time.

In another preferred preparation for the composition of the present invention, both the covalently conjugated protein or peptide and the remaining components of the composition may be separately packaged in different containers (*i.e.,* syringes). The contents of the containers may be combined with the remaining components of the composition immediately prior to administration to an implant site in the body of a subject.

According to the present invention, the composition preferably is a uniform solution or homogeneous suspension. The maintenance of the uniformity of the formulation prior to administration is critical to obtain consistent depot system for controlled release delivery of bioactive substances. Practically, the uniformity of the formulation has to be maintained for at least one hour to allow reproducible preparation of the formulation and formation of the depot system *in situ.* The uniformity as used herein is determined by measuring the ratio or distribution the protein or peptide in the top portion and bottom portion of the formulation in a 5 ml glass test tube. If the ratio equals to 1.0, the formulation has a perfect uniformity. If the ratio is smaller than 1.0, it indicates that phase separation occurs. Preferably, the composition maintains a uniformity of 0.9 for at least one hour, more preferably the composition maintains a uniformity of 0.9 for at least 24 hours, most preferably the composition maintains a uniformity of 0.9 for at least 7 days.

According to the present invention, the composition contains from about 10% to about 99.5% of the hydrophobic, non-polymeric material, preferably between 25% and 95% by weight relative to the total weight of the composition. The composition also includes about 0% to about 50% of a pharmaceutically acceptable solvent, about 0.1% to about 40% of a protein or peptide. The composition further contains about 1% to about 25% of an additive.

In a preferred embodiment, sucrose acetate isobutyrate (SAIB) is used as the hydrophobic, non-polymeric carrier material and NMP is selected as the solvent. The peptide or protein is selected from a group consisting of octreotide, or glycagon like peptide-1 (GLP-1). The protein or peptide is preferably conjugated with an amphiphilic molecule. The resulting conjugate can be combined with the SAIB/NMP solution to form a sustained delivery formulation.

According to the present invention, the composition described herein can be administered to the body of a subject where sustained release delivery of a protein or peptide is desired. As used herein, the term "subject" is intended to include warm-blooded animals, preferably mammals, most preferably humans.

As used herein, the term "administered" is intended to refer to dispensing, delivering or applying a composition (e.g., pharmaceutical formulation) to a subject by any suitable route for delivery of the composition to the desired location in the subject. The composition can be administered to a subject topically, by injection subcutaneously, intramuscularly, intraperitoneally, or intradermally, and by oral, rectal, vaginal, or nasal administration to provide the desired dosage of a protein or peptide based on the known parameters for treatment of the various diseases with the proteins or peptides.

The term "controlled, sustained release delivery", as used herein, includes, for example, continual delivery of a protein or peptide *in vivo* over a period of time following administration, preferably at least several days to weeks or months. Controlled, sustained release delivery of the protein or peptide can be demonstrated, for example, by the continued therapeutic effect of the agent over time (e.g., for octreotide, sustained delivery of the peptide can be demonstrated by continued IGF-1 suppression over time). Alternatively, sustained delivery of the octreotide may be demonstrated by detecting the presence of the peptide *in vivo* over time.

In this application, the various embodiments set forth in the claims for the instant liquid pharmaceutical compositions are also envisioned, mutatis mutandis, for the instant methods for forming such compositions and the instant methods for forming implants.

### EXAMPLES:

The following examples illustrate the compositions and methods of the present invention. The following examples should be merely interpreted to teach how to make the useful drug delivery systems according to the present invention.

### Example 1. Preparation of Palmitoyl-Octreotide (PAL-OCT)

50 mg of octreotide acetate was dissolved in 1 mL of anhydrous DMSO containing 100 µL triethylamine (TEA). 40.2 mg of palmitic acid N-hydroxysuccinimide ester (Mw 353.50) was dissolved in 3 mL anhydrous DMSO and added to the peptide solution. The reaction was allowed to proceed for 3 hours at room temperature. The mixture was poured into diethylether to precipitate palmitoylated octreotide. The precipitate was washed with diethylether twice and then dried under vacuum. The resulting acylated peptide was in the form of a white powder.

### Example 2. Preparation of Palmitoyl-Octreotide (PAL-OCT)

50 mg of octreotide acetate was dissolved in 1000 µL of anhydrous DMSO containing 100 µL TEA. 17.1 mg of palmitic acid N-hydroxysuccinimide ester (Mw 353.50) was dissolved in 3 mL anhydrous DMSO and added by direct injection to the peptide solution. The reaction was allowed to proceed overnight at room temperature. The mixture was poured into diethylether to precipitate palmitoylated octreotide. The precipitate was washed with diethylether twice and then dried under vacuum. The resulting acylated peptide was in the form of white powder.

### Example 3. Preparation of Decanal-Octreotide (DCL-OCT)

50 mg of octreotide was dissolved in 2 mL of 20 mM sodium cyanoborohydride (Mw 62.84, NaCNBH₃) (2.51 mg) solution in 0.1 M acetate buffer at pH 5. 13.7 mg of Decanal (Mw 156.27) (OCT:DCL = 1:2) was added by direct injection to the peptide solution. The reaction was allowed to proceed overnight at 4 °C. The mixture was separated by centrifugation. The precipitated DCL-OCT was freeze-dried.

### Example 4. Preparation of Palmitoyl-Lysozyme (PAL-Lyz, 3:1)

302 mg of Lysozyme (Mw 14,500) was dissolved in 1000 µL of anhydrous DMSO containing 200 µL TEA. 18.25 mg of Palmitic acid N-hydroxysuccinimide ester (Mw 353.50) was dissolved in 3 mL anhydrous DMSO and added by direct injection to the protein solution. The reaction was allowed to proceed for overnight at RT. The PAL-Lyz was precipitated in diethylether and the final product was freeze-dried after removing the organic solvent.

### Example 6. Preparation of Palmitoyl-Lysozyme (PAL-Lyz, 5:1)

50 mg of lysozyme (Mw 14,500) was dissolved in water and pH was adjusted to 9.58. The solution was freeze-dried. Then the dried powder was dissolved in 3 mL DMSO. Then 322 µL of 20 mg/mL solution of palmitic acid N-hydroxysuccinimide ester (Mw 353.50) in anhydrous DMSO was added by direct injection to the protein solution. The reaction was allowed to proceed overnight at 4 °C. The PAL-Lyz was precipitated in diethylether and the final product was freeze-dried after removing the organic solvent.

### Example 7. Preparation of Palmitoyl-Lysozyme (PAL-Lyz, 13:1)

50 mg of lysozyme (Mw 14,500) was dissolved in water and pH was adjusted to 9.58. The solution was freeze-dried. Then the dried powder was dissolved in 3 mL DMSO. Then 799 µL of 20 mg/mL solution of palmitic acid N-hydroxysuccinimide ester (Mw 353.50) in anhydrous DMSO was added by direct injection to the protein solution. The reaction was allowed to proceed overnight at 4 °C. The PAL-Lyz was precipitated in diethylether and the final product was freeze-dried after removing the organic solvent.

### Example 8. Preparation of Palmitoyl-Lysozyme (PAL-Lyz)

Lysozyme is added to PAL-NHS in PBS (pH 8.0) containing 2% deoxycholate (DOC). The mixture is incubated at 37 °C for 6 hours. The mixture is centrifuged to remove the unreacted PAL-NHS. The product is dialyzed against PBS containing 0.15% DOC for 48 h (PAL-NHS:Lysozyme=15:1).

### Example 9. Preparation of Monopalmityl Poly(ethylene glycol)-Butyraldehyde, Diethyl Acetal.

A mixture of monopalmityl poly(ethylene glycol) (average Mn ∼1124) (5.0 g, 4.45 mmoles) and toluene (75 mL) was azeotropically dried by distilling off toluene under reduced pressure. The dried monopalmityl poly(ethylene glycol), was dissolved in anhydrous toluene (50 mL) to which was added a 20% (w/w) solution of potassium *tert*-butoxide in THF (4.0 ml, 6.6 mmoles) and 4-chlorobutyraldehyde diethyl acetal (0.96 g, 5.3 mmoles, MW 180.67). The mixture was stirred at 100-105 °C overnight under an argon atmosphere. After cooling to room temperature, the mixture was filtered and added to 150 ml ethyl ether at 0-5 °C. The precipitated product was filtered off and dried under reduced pressure.

### Example 10. Conjugation of Octreotide at N-terminal Amine Group with Monopalmityl Poly(ethylene glycol) (PAL-PEG-BA-OCT)

In a typical preparation, 201.6 mg of monopalmityl poly(ethylene glycol)-butyraldehyde, diethyl acetal (PAL-PEG-BADA) was dissolved in 10 mL of 0.1 M phosphoric acid (pH 2.1) and the resulting solution was heated at 50 °C for 1 h then cooled to room temperature. The pH of the solution was adjusted to 5.5 with 1 N NaOH and the resulting solution was added to a solution of 195.3 mg of octreotide in 3.5 mL of 0.1 M sodium phosphate buffer (pH 5.5). After 1 h, 18.9 mg of NaCNBH₃ was added to have a concentration of 20 mM. The reaction was continued overnight at room temperature. Then the reaction mixture was either dialyzed with a membrane having a MW cutoff of 2000 daltons or loaded on a preparative HPLC with a C-18 column. The purified conjugated octreotide was primarily a single compound with one primary amine (lysine), and one secondary amine (N-terminal).

### Example 11. Preparation and in vitro characterization of formulations containing octreotide

Octreotide acetate (OCT-Ac) and octreotide conjugate (PAL-PEG-BA-OCT) powders were dissolved in NMP. Then the solutions containing various forms of octreotide were thoroughly mixed with SAIB solution in NMP (90% w/w). The octreotide content was about 6% for all formulations as shown in Table 1.

**Table 1. Formulations containing octreotide**

| **Formulation** | **Peptides (mg)** | **SAIB (mg)** | **NMP (mg)** | **Peptide Content (%)** |
|---|---|---|---|---|
| OCT-Ac/SAIB/NMP | 65.9 | 653.5 | 281.8 | 6% |
| PAL-PEG-BA-OCT/SAIB/NMP | 103.9 | 500.9 | 263. | 6% |

The formulations as prepared above were allowed to stand at room temperature (∼22° C). At predefined time points, the appearance of the formulations was recorded and an aliquot of each of both the supernatant and bottom composition was collected and analyzed by HPLC for octreotide content. The physical stability of the formulations was determined by direct observation or using the ratio of the content of the octreotide in the supernatant to that in the bottom composition. If the ratio equals to 1.0, the formulation is uniform and stable. If the ratio is smaller than 1.0, that indicates that phase separation occurs.

When the OCT-Ac was combined with SAIB/NMP solution, phase separation occurred immediately. Chunky solid precipitates were observed and an inhomogeneous formulation was obtained. The chunky aggregates were precipitated to form a yellow sticky phase at the bottom over time. This inhomogeneous formulation will block needles and is not suitable for injection. When PAL-PEG-BA-OCT was combined with SAIB/NMP solution, a clear solution was obtained and suitable for injection.

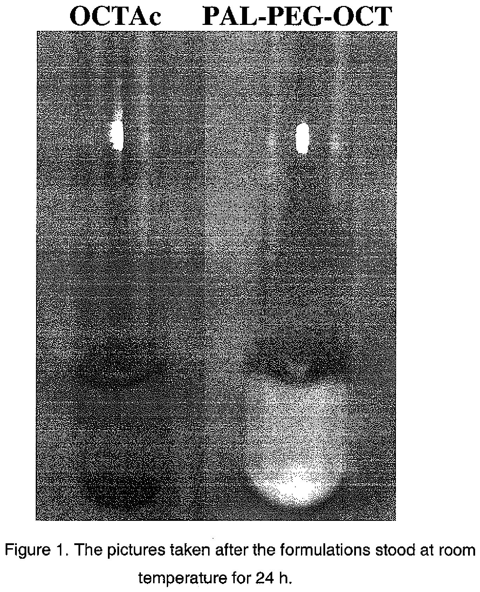

Figure 1 shows the pictures taken after the formulations stood at room temperature for 24 h. An apparent phase separation occurred for the formulations containing OCT-Ac, but not for the one containing PAL-PEG-BA-OCT. As shown in Table 2, the concentration of the octreotide in the bottom phase was about 20 folds of that of the octreotide in the upper phase after two days. This ratio decreased to less than 0.03 after 5 days. Surprisingly, no phase separation was observed for the formulation containing PAL-PEG-BA-OCT. In addition, slightly more NMP was observed for the formulations containing OCT-Ac in the bottom phase.

**Table 2: The ratio of the octreotide and NMP in the top phase to those in the bottom phase after stood at room temperature for two days**

| **Formulation** | **OCT Ratio Top/Bottom** | **NMP Ratio Top/Bottom** |
|---|---|---|
| OCT-Ac/SAIB/NMP | 0.05 | 0.93 |
| PAL-PEG-BA-OCT/SAIB/NMP | 1.00 | 1.00 |

In addition, it was found that octreotide was not stable in the formulation containing OCT-Ac. As shown in Table 3, the generation of the impurities of the octreotide occurred as soon as the components were combined. After two hours, about 4% of the octreotide was degraded or reacted. More than half of the octreotide in the supernatant and more than 20% of the octreotide in the bottom phase were degraded after 7 days, indicating that the system is not suitable for the sustained delivery of the peptide. However, it was unexpectedly found that little or no degradation of the octreotide was detected from the formulations containing PAL-PEG-BA-OCT even after three months at room temperature (Table 3). No phase separation was observed in the formulations containing PAL-PEG-BA-OCT.

**Table 3: Purity of the octreotide in formulations at room temperature over time**

| **Time (day)** | OCT-Ac/SAIB/NMP | PAL-PEG-BA-OCT/SAIB/NMP |
|---|---|---|
| 0.08 | 96.1 | 100 |
| 1 | 90.8 | 99.9 |
| 2 | 71.9 | ND |
| 5 | 48.7 | ND |
| 7 | 41.0 | 99.8 |
| 112 | 33.9 | 100 |

### Example 12. In vitro release of octreotide from various formulations

Formulations were prepared by mixing octreotide acetate (OCT-Ac) and octreotide conjugate (PAL-PEG-BA-OCT) powders with SAIB solution in NMP (90% w/w). The octreotide content was about 6% in each formulation as shown in Table 4.

**Table 4. Formulations containing octreotide**

| **Formulation** | **Peptides (mg)** | **SAIB/NMP (mg)** | **Peptide Content (%)** |
|---|---|---|---|
| OCT-Ac/SAIB/NMP | 68.5 | 970.7 | 6% |
| PAL-PEG-BA-OCT/SAIB/NMP | 113.8 | 834.2 | 6% |

Due to the physical instability of the formulation containing OCT-Ac, the *in vitro* release was investigated immediately after the preparation of the formulations. An aliquot of the suspension was used for the *in vitro* release. About 0.1 mL of each formulation containing octreotide was injected into 3 mL of releasing buffer (PBS 7.4, containing 0.1% sodium azide) in a 4 mL glass vial. The vials were incubated at 37°C and sampled at various time points. At each time point, 2 mL of release medium was removed and 2 mL of fresh release medium added. The collected samples were analyzed for peptide concentration and integrity by HPLC using an YMC-Pack ODS-120A column. Triplicate samples were used for each formulation.

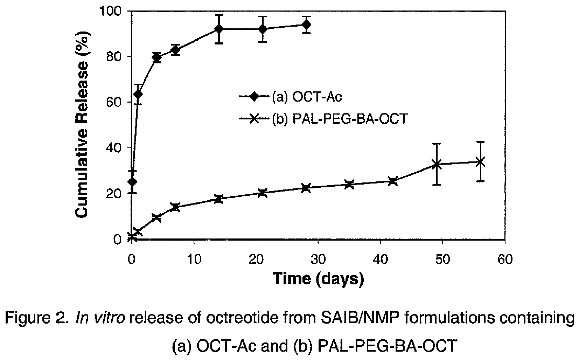

As shown in Figure 2, the release of OCT from formulation containing OCT-Ac showed a very high initial burst release. More than 60% of the octreotide was released within 24 hours and more than 90% of the octreotide was released after two weeks. However, surprisingly, the release of OCT from formulations containing PAL-PEG-BA-OCT did not show much initial burst release. Less than 5% of the octreotide was released within 24 hours from formulation containing PAL-PEG-BA-OCT followed by a gradual release over time.

### Example 13. Preparation of Polyethylene Glycol Conjugated GM-CSF

GM-CSF can be covalently conjugated to polyethylene glycol (PEG) as follows: 100 mg of GM-CSF is dissolved in 10 ml pH 7.5 phosphate buffer, at room temperature. 100 mg tresyl-monomethoxy-polyethylene glycol (MW=5000 daltons) is then added, and the mixture stirred for 1 hour. The unreacted GM-CSF and pegylated GM-CSF fractions are isolated from unreacted tresyl-monomethoxy-polyethylene glycol by gel chromatography. The pegylated GM-CSF is then dialyzed into 100 mM Tris buffer and adjusted to a concentration of 50 mg/ml.

### Example 14. Preparation of Polyethylene Glycol-Conjugated Human Insulin (PEG-Insulin)

Human insulin was covalently modified with polyethylene glycol as follows: 116 mg of recombinant human insulin is dissolved in 4 mL anhydrous DMSO containing 200 uL TEA. 1 g of mPEG(5000)-SPA is dissolved in 10 mL anhydrous DMSO and added to the insulin solution by direct injection. The reaction proceeds overnight (>10 hours) at room temperature or until >90% of the protein is pegylated. The unreacted PEG and pegylated insulin are isolated by precipitation twice from ether. The final product is analyzed by RP-HPLC.

### Example 15. Preparation of Pegylated Lysozyme (PEG-Lyz)

Lysozyme solution (0.4% w/v, 5 mL) in borate buffer (20 mM, pH 9.0) was cooled down to 4°C. 209 mg MPEG-SS (MW 2000, 11 molar excess) was slowly added to the protein solution. The reaction was allowed to proceed at 4°C overnight with an end to end rotation, and stopped by the addition of 10 molar excess glycine. The reaction mixture was dialyzed using 3500 Dalton pore size membrane against the DI water. The dialyzed sample was lyophilized in water without any addition of additives and stored at-20°C.

The invention is not limited by the embodiments described above which are presented merely as examples and can be modified in various ways within the scope of protection defined by the appended patent claims.

## Claims

1. An injectable composition for sustained release of a protein or peptide, comprising:
(a) a hydrophobic non-polymeric carrier material formed from polyol having less than 20 hydroxyl groups that are esterified with carboxylic acids, wherein the hydrophobic, non-polymeric material is a liquid that has a viscosity of at least 5,000cP at 37°C that does not crystallize neat under ambient or physiological conditions;
(b) a water miscible pharmaceutically acceptable organic solvent having a miscibility of at least 1% by weight in water at 25°C; and
(c) a protein or peptide covalently conjugated with amphiphilic molecule(s)
having the following general structures:
L-S-(OC₂H₄)ₘOH (Formula 1)
wherein L is the lipophilic moiety preferably selected from C4-36-alkyl, C4-36-alkenyl, C4-36-alkadienyl, tocopherol and steroidal residues, and wherein S is a linker selected from a group of an ester group, amide group, secondary or tertiary amine group, carbamate group, sulfonate group, sulfate group, phosphate group, phosphonate group, or ether group, wherein m ranges from 1 to 1000, the amphiphilic molecule(s) comprises (1) a linear polyethylene glycol moiety and (2) a lipophilic moiety, wherein the protein or peptide, the polyethylene glycol and the lipophilic moiety are conformationally arranged to have the lipophilic moiety exteriorly available for interaction with lipophilic environment or cell membranes.

2. The composition of claim **1,** wherein the hydrophobic, non-polymeric carrier material has low crystallinity and non-polar characteristics.

3. The composition of claim **1,** wherein the carboxylic acids include organic acids having more than two carbons, such as fatty acids.

4. The composition of claim **1,** wherein the hydrophobic, non-polymeric carrier material is sucrose acetate isobutyrate (SAIB).

5. The composition of claim **1,** wherein the pharmaceutically acceptable solvent is selected from the group consisting of benzyl alcohol, caprolactam, caprolactone, dimethylsulfoxide (DMSO), ethanol, ethyl lactate, glycerol, glycerol formal, glycofurol (tetraglycol), N-methyl-2-pyrrolidone (NMP), polyethylene glycol, PEG-300, PEG-400, methoxy polyethylene glycol, mPEG-350, alkoxy polyethylene glycol, propylene carbonate, triacetin, triethyl citrate, and combinations thereof.

6. The composition of claim 1, wherein the protein or peptide is selected from the group consisting of oxytocin, vasopressin, adrenocorticotropic hormone (ACTH), epidermal growth factor (EGF), platelet-derived growth factor (PDGF), prolactin, luteinising hormone, luteinizing hormone releasing hormone (LHRH), LHRH agonists, LHRH antagonists, growth hormones (including human, porcine, and bovine), growth hormone releasing factor, insulin, insulin-like growth factors (IGF-I, IGF-II), erythropoietin (including all proteins with erythropoietic activity), somatostatins, glucagon, interleukin, interferon-α, interferon-β, interferon-γ, gastrin, tetragastrin, pentagastrin, urogastrone, secretin, calcitonin, enkephalins, endorphins, angiotensins, thyrotropin releasing hormone (TRH), tumor necrosis factor (TNF), parathyroid hormone (PTH), nerve growth factor (NGF), granulocyte-colony stimulating factor (G-CSF), granulocyte macrophage-colony stimulating factor (GM-CSF), macrophage-colony stimulating factor (M-CSF), heparinase, vascular endothelial growth factor (VEG-F), bone morphogenic protein (BMP), hANP, glucagon-like peptide (GLP-1, GLP-2), exenatide (exendin-3, exendin-4, etc.), peptide YY (PYY), renin, bradykinin, bacitracins, polymyxins, colistins, tyrocidine, gramicidins, cyclosporins (which includes synthetic analogues and pharmacologically active fragments thereof), enzymes, cytokines, antibodies, vaccines, antibiotics, glycoproteins, follicle stimulating hormone, kyotorphin, tuftsin, thymopoietin, thymosin, thymostimulin, thymic humoral factor, serum thymic factor, colony stimulating factors, motilin, bombesin, dynorphin, neurotensin, cerulein, urokinase, kallikrein, substance P analogues and antagonists, angiotensin II, blood coagulation factor VII and IX, lysozyme, melanocyte stimulating hormone, thyroid hormone releasing hormone, thyroid stimulating hormone, pancreozymin, cholecystokinin, human placental lactogen, thrombopoietin (TPO), human chorionic gonadotrophin, protein synthesis stimulating peptide, gastric inhibitory peptide, vasoactive intestinal peptide, and synthetic analogues and modifications and pharmacologically-active fragments and pharmaceutically acceptable salts thereof.

7. The composition of claim **1,** wherein the lipophilic moieties of the amphiphilic molecule include lauryl, palmitoyl, stearoyl, oleyl, eicosanoyl, and docosanoyl.

8. The composition of claim **1,** wherein the molar ratio of the protein or peptide to the amphiphilic molecule in the conjugate varies from 1:1 to 1:10 according to the nature of the protein or peptide.

9. The composition according to claim **1,** wherein the hydrophobic, non-polymeric carrier material is sucrose acetate isobutyrate (SAIB), wherein the pharmaceutically acceptable solvent is N-methyl-2-pyrrolidone (NMP).

10. The composition according to claim 1, wherein the bioactive substance is selected from the group consisting of octreotide or glucagon like peptide-1 (GLP-1).

## Patentansprüche

1. Injizierbare Zusammensetzung zur anhaltenden Freisetzung eines Proteins oder Peptids, umfassend:
(a) ein hydrophobes, nichtpolymeres Trägermaterial, gebildet aus einem Polyol mit weniger als 20 Hydroxylgruppen, die mit Carbonsäuren verestert sind, wobei das hydrophobe, nichtpolymere Material eine Flüssigkeit ist, die eine Viskosität von mindestens 5000 cP bei 37 °C aufweist, die rein unter Umgebungsbedingungen oder physiologischen Bedingungen nicht kristallisiert;
(b) ein mit Wasser mischbares, pharmazeutisch akzeptables organisches Lösungsmittel mit einer Mischbarkeit von mindestens 1 Gew.-% in Wasser bei 25 °C; und
(c) ein Protein oder Peptid, kovalent konjugiert mit (einem) amphiphilen Molekül(en) mit den folgenden allgemeinen Strukturen:
L-S-(OC₂H₄)ₘOH (Formel 1),
wobei L der lipophile Teil, vorzugsweise ausgewählt aus C₄₋₃₆-Alkyl, C₄₋₃₆-Alkenyl, C₄₋₃₆-Alkadienyl, Tocopherol und Steroidresten, ist, und wobei S ein Linker, ausgewählt aus einer Gruppe aus einer Estergruppe, Amidgruppe, sekundären oder tertiären Amingruppe, Carbamatgruppe, Sulfonatgruppe, Sulfatgruppe, Phosphatgruppe, Phosphonatgruppe oder Ethergruppe, ist, wobei m von 1 bis 1000 reicht, das/die amphiphile(n) Molekül(e) (1) einen linearen Polyethylenglykolteil und (2) einen lipophilen Teil umfasst/umfassen, wobei das Protein oder Peptid, das Polyethylenglykol und der lipophile Teil konformativ derart angeordnet sind, dass der lipophile Teil für die Wechselwirkung mit einer lipophilen Umgebung oder Zellmembranen außenliegend zugänglich ist.

2. Zusammensetzung nach Anspruch **1,** wobei das hydrophobe, nichtpolymere Trägermaterial eine geringe Kristallinität und unpolare Eigenschaften aufweist.

3. Zusammensetzung nach Anspruch **1,** wobei die Carbonsäuren organische Säuren mit mehr als zwei Kohlenstoffatomen, wie Fettsäuren, umfassen.

4. Zusammensetzung nach Anspruch **1,** wobei das hydrophobe, nichtpolymere Trägermaterial Saccharoseacetatisobutyrat (SAIB) ist.

5. Zusammensetzung nach Anspruch **1,** wobei das pharmazeutisch akzeptable Lösungsmittel ausgewählt ist aus der Gruppe bestehend aus Benzylalkohol, Caprolactam, Caprolacton, Dimethylsulfoxid (DMSO), Ethanol, Ethyllactat, Glycerin, Glycerinformal, Glykofurol (Tetraglykol), N-Methyl-2-pyrrolidon (NMP), Polyethylenglykol, PEG-300, PEG-400, Methoxypolyethylenglykol, mPEG-350, Alkoxypolyethylenglykol, Propylencarbonat, Triacetin, Triethylcitrat, und Kombinationen davon.

6. Zusammensetzung nach Anspruch **1,** wobei das Protein oder Peptid ausgewählt ist aus der Gruppe bestehend aus Oxytocin, Vasopressin, adrenocorticotropem Hormon (ACTH), epidermalem Wachstumsfaktor (EGF), Thrombozyten-Wachstumsfaktor (PDGF), Prolactin, luteinisierendem Hormon, luteinisierendes Hormon freisetzendem Hormon (LHRH), LHRH-Agonisten, LHRH-Antagonisten, Wachstumshormonen (einschließlich humaner, porciner und boviner Wachstumshormone), Wachstumshormon freisetzendem Faktor, Insulin, insulinähnlichen Wachstumsfaktoren (IGF-I, IGF-II), Erythropoetin (einschließlich aller Proteine mit erythropoetischer Aktivität), Somatostatinen, Glucagon, Interleukin, Interferon-α, Interferon-β, Interferon-γ, Gastrin, Tetragastrin, Pentagastrin, Urogastron, Sekretin, Calcitonin, Enkephalinen, Endorphinen, Angiotensinen, Thyrotropin freisetzendem Hormon (TRH), Tumor-Nekrose-Faktor (TNF), Parathormon (PTH), Nervenwachstumsfaktor (NGF), Granulozyten-Kolonie-stimulierendem Faktor (G-CSF), Granulozyten-Makrophagen-Kolonie-stimulierendem Faktor (GM-CSF), Makrophagen-Kolonie-stimulierendem Faktor (M-CSF), Heparinase, vaskulärem endothelialem Wachstumsfaktor (VEGF), Knochenmorphogenese-Protein (BMP), hANP, glucagonähnlichem Peptid (GLP-1, GLP-2), Exenatid (Exendin-3, Exendin-4 usw.), Peptid YY (PYY), Renin, Bradykinin, Bacitracinen, Polymyxinen, Colistinen, Tyrocidin, Gramicidinen, Cyclosporinen (was synthetische Analoga und pharmakologisch wirksame Fragmente davon einschließt), Enzymen, Cytokinen, Antikörpern, Impfstoffen, Antibiotika, Glykoproteinen, Follikel-stimulierendem Hormon, Kyotorphin, Tuftsin, Thymopoetin, Thymosin, Thymostimulin, humoralem Thymusfaktor, Serum-Thymusfaktor, Kolonie-stimulierenden Faktoren, Motilin, Bombesin, Dynorphin, Neurotensin, Cerulein, Urokinase, Kallikrein, Substanz-P-Analoga und Substanz-P-Antagonisten, Angiotensin II, Blutgerinnungsfaktor VII und Blutgerinnungsfaktor IX, Lysozym, Melanozytenstimulierendem Hormon, Schilddrüsenhormon freisetzendem Hormon, Thyreotropin, Pankreozymin, Cholecystokinin, humanem plazentarem Lactogen, Thrombopoetin (TPO), humanem Choriongonadotropin, Proteinsynthese-stimulierendem Peptid, gastrischem inhibitorischem Peptid, vasoaktivem intestinalem Peptid, und synthetischen Analoga und Modifikationen und pharmakologisch wirksamen Fragmenten und pharmazeutisch akzeptablen Salzen davon.

7. Zusammensetzung nach Anspruch **1,** wobei die lipophilen Teile des amphiphilen Moleküls Lauryl, Palmitoyl, Stearoyl, Oleyl, Eicosanoyl und Docosanoyl umfassen.

8. Zusammensetzung nach Anspruch **1,** wobei das Molverhältnis des Proteins oder Peptids zu dem amphiphilen Molekül in dem Konjugat je nach Beschaffenheit des Proteins oder Peptids von 1:1 bis 1:10 variiert.

9. Zusammensetzung nach Anspruch **1,** wobei das hydrophobe, nichtpolymere Trägermaterial Saccharoseacetatisobutyrat (SAIB) ist, wobei das pharmazeutisch akzeptable Lösungsmittel N-Methyl-2-pyrrolidon (NMP) ist.

10. Zusammensetzung nach Anspruch **1,** wobei die bioaktive Substanz ausgewählt ist aus der Gruppe bestehend aus Octreotid oder glucagonähnlichem Peptid 1 (GLP-1).

## Revendications

1. Composition injectable pour la libération prolongée d'une protéine ou d'un peptide, comprenant :
(a) un matériau véhicule non polymère hydrophobe formé à partir d'un polyol ayant moins de 20 groupements hydroxyles qui sont estérifiés par des acides carboxyliques, où le matériau non polymère hydrophobe est un liquide qui a une viscosité de 5 000 cP au moins à 37 °C et qui ne cristallise pas à l'état non dilué dans des conditions ambiantes ou physiologiques ;
(b) un solvant organique pharmaceutiquement acceptable miscible à l'eau qui a une miscibilité de 1 % en poids au moins dans l'eau à 25 °C ; et
(c) une protéine ou un peptide conjugué d'une manière covalente avec une ou des molécules amphiphiles ayant les structures générales suivantes :
L-S-(OC₂H₄)ₘOH (Formule 1)
où L est la fraction lipophile, qui est de préférence sélectionnée parmi un alkyle en C4-36, un alcényle en C4-36, un alkadiényle en C4-36, un tocophérol et des résidus stéroïdiens, S est un coupleur sélectionné dans le groupe consistant en un groupement ester, un groupement amide, un groupement amine secondaire ou tertiaire, un groupement carbamate, un groupement sulfonate, un groupement sulfate, un groupement phosphate, un groupement phosphonate ou un groupement éther et m va de 1 à 1 000, la ou les molécules amphiphiles comprenant (1) une fraction polyéthylène glycol linéaire et (2) une fraction lipophile, où la protéine ou le peptide, le polyéthylène glycol et la fraction lipophile sont agencés sur le plan conformationnel d'une manière telle que la fraction lipophile est située à l'extérieur et peut ainsi interagir avec un environnement lipophile ou des membranes cellulaires.

2. Composition de la revendication **1,** où le matériau véhicule non polymère hydrophobe a une faible cristallinité et des caractéristiques non polaires.

3. Composition de la revendication **1,** où les acides carboxyliques incluent des acides organiques ayant plus de deux carbones, comme des acides gras.

4. Composition de la revendication **1,** où le matériau véhicule non polymère hydrophobe est l'acétate-isobutyrate de saccharose (SAIB).

5. Composition de la revendication **1,** où le solvant pharmaceutiquement acceptable est sélectionné dans le groupe consistant en les suivants : alcool benzylique, caprolactame, caprolactone, diméthylsulfoxyde (DMSO), éthanol, lactate éthylique, glycérol, glycérol formal, glycofurol (tétraglycol), N-méthyl-2-pyrrolidone (NMP), polyéthylène glycol, PEG-300, PEG-400, méthoxypolyéthylène glycol, mPEG-350, alkoxypolyéthylène glycol, carbonate de propylène, triacétine, citrate de triéthyle et combinaisons de ceux-ci.

6. Composition de la revendication **1,** où la protéine ou le peptide est sélectionné dans le groupe consistant en les suivants : oxytocine, vasopressine, hormone adrénocorticotrope (ACTH), facteur de croissance épidermique (EGF), facteur de croissance dérivé des plaquettes (PDGF), prolactine, lutéinostimuline, hormone de libération de la lutéinostimuline (LH-RH), agonistes de la LH-RH, antagonistes de la LH-RH, hormones de croissance (y compris humaines, porcines et bovines), facteur de libération de l'hormone de croissance, insuline, facteurs de croissance semblables à l'insuline (IGF-I, IGF-II), érythropoïétine (y compris toutes les protéines à activité érythropoïétique), somatostatines, glucagon, interleukine, interféron-α, interféron-β, interféron-γ, gastrine, tétragastrine, pentagastrine, urogastrone, sécrétine, calcitonine, encéphalines, endorphines, angiotensines, hormone de libération de la thyréostimuline (TRH), facteur de nécrose tumorale (TNF), parathormone (PTH), facteur de croissance nerveuse (NGF), facteur de stimulation des colonies de granulocytes (G-CSF), facteur de stimulation des colonies de granulocytes-macrophages (GM-CSF), facteur de stimulation des colonies de macrophages (M-CSF), héparinase, facteur de croissance endothéliale vasculaire (VEGF), protéine morphogénétique osseuse (BMP), hANP, glucagon-like peptide (GLP-1, GLP-2), exénatide (exendine-3, exendine-4, etc.), peptide YY (PYY), rénine, bradykinine, bacitracines, polymyxines, colistines, tyrocidine, gramicidines, cyclosporines (qui incluent les analogues de synthèse et fragments pharmacologiquement actifs de ceux-ci), enzymes, cytokines, anticorps, vaccins, antibiotiques, glycoprotéines, folliculostimuline, kyotorphine, tuftsine, thymopoïétine, thymosine, thymostimuline, facteur thymique humoral, facteur thymique sérique, facteurs de stimulation de colonies, motiline, bombésine, dynorphine, neurotensine, céruléine, urokinase, kallikréine, analogues et antagonistes de la substance P, angiotensine II, facteurs de la coagulation sanguine VII et IX, lysozyme, hormone mélanotrope, hormone de libération des hormones thyroïdiennes, thyréostimuline, pancréozymine, cholécystokinine, lactogène placentaire humain, thrombopoïétine (TPO), gonadotrophine chorionique humaine, peptide stimulant la synthèse de protéines, peptide inhibiteur gastrique, peptide intestinal vasoactif et analogues et modifications de synthèse, fragments pharmacologiquement actifs et sels pharmaceutiquement acceptables de ceux-ci.

7. Composition de la revendication **1,** où les fractions liphophiles de la molécule amphiphile incluent les suivantes : lauryle, palmitoyle, stéaroyle, oléyle, éicosanoyle et docosanoyle.

8. Composition de la revendication **1,** où le rapport molaire entre la protéine ou le peptide et la molécule amphiphile du conjugué varie de 1:1 à 1:10 en fonction de la nature de la protéine ou du peptide.

9. Composition de la revendication **1,** où le matériau véhicule non polymère hydrophobe est l'acétate-isobutyrate de saccharose (SAIB) et où le solvant pharmaceutiquement acceptable est la N-méthyl-2-pyrrolidone (NMP).

10. Composition selon la revendication **1,** où la substance bioactive est sélectionnée dans le groupe consistant en l'octréotide ou le glucagon-like peptide-1 (GLP-1).
